Europäisches Patentamt

European Patent Office

Office européen des brevets

(11) Publication number: **0 263 659**
**A2**

# (12) EUROPEAN PATENT APPLICATION

(21) Application number: **87308796.9**

(51) Int. Cl.⁴: **C 12 N 5/00**

(22) Date of filing: **05.10.87**

(30) Priority: **06.10.86 US 915676**

(43) Date of publication of application:
**13.04.88 Bulletin 88/15**

(84) Designated Contracting States:
**AT BE CH DE ES FR GB GR IT LI LU NL SE**

(71) Applicant: **Winter, Susan C.**
**6641 N. Forkner**
**Fresno California 93711 (US)**

**Szabo, Steven**
**2220 Villa Avenue no. 2**
**Clovis California 93612 (US)**

**Tsai, Joseph Chien-Chih**
**756 E. Serena**
**Fresno California 93710 (US)**

(72) Inventor: **Winter, Susan C.**
**6641 N. Forkner**
**Fresno California 93711 (US)**

**Szabo, Steven**
**2220 Villa Avenue no. 2**
**Clovis California 93612 (US)**

**Tsai, Joseph Chien-Chih**
**756 E. Serena**
**Fresno California 93710 (US)**

(74) Representative: **Harrison, David Christopher et al**
**MEWBURN ELLIS & CO 2/3 Cursitor Street**
**London EC4A 1BQ (GB)**

(54) Tissue culture media containing L-carnitine.

(57) A cell culture medium and a method of culturing animal cells are described in which L-carnitine is incorporated into a cell culture medium suitable for sustaining the growth of an animal cell line. Improved growth of the cell line is obtained.

EP 0 263 659 A2

**Description**

## TISSUE CULTURE MEDIA CONTAINING L-CARNITINE

This invention relates to tissue culture media, particularly media used for the in vitro culture of human cell lines.

Numerous media suitable for use in animal cell culture exist having more or less defined compositions. For example, a list of over twenty different cell culture media formulations is presented in the America Type Culture Collection Media Handbook, first edition, 1984, R. Cote, ed., American Type Culture Collection, Rockville, Maryland. Included in this general reference work is a list of fifty-eight references (on pages 83-84) that describe a number of aspects relating to cell culture including basic references to specific media, tissue dispersion, cell harvests, studies directed toward totally synthetic media, nutritional needs of mammalian cells in tissue culture, and optimization of the growth of numerous types of cells.

The media described in this publication and elsewhere are combinations of salts, energy sources (usually glucose or another carbohydrate), vitamins, and various indicators, such as pH indicators, for maintaining proper conditions for growth in the media. Many of the media contain proteins, hormones of various types necessary for a specific cell line or tissue, bacteriocides, and the like. Because many of the media contain either serum or serum proteins, they are not completely defined. The exact role of serum in animal cell cultures is still unknown at the present time, as it is thought that certain macromolecules, proteins, hormones, or entrapped trace elements may be responsible for the beneficial effects of the serum components.

Despite numerous advances in understanding cell culture media, the art of tissue culture remains largely empirical. There remains a need for identification of other components present in serum that have advantageous effects on the growth of cell lines and tissue cultures in order to maximize cell growth, particularly in commercial areas such as hybridoma technology and clinical analysis.

SUMMARY OF THE INVENTION

The present invention provides a method of improving the growth of an animal cell line in a cell culture medium, along with the improved medium utilized in the process, in which L-carnitine is added to the culture medium. Improved growth, including both improved rate of growth and better initiation of cultures, have been demonstrated for a number of different cell lines and cell culture media.

DESCRIPTION OF PREFERRED EMBODIMENTS

L-carnitine, also known as gamma-trimethylamino-beta-hydroxybutyrate, is a normal endogenous intermediary metabolite present in blood, urine, and all cells of animals. Prior to the present invention, carnitine was known to function as a transport protein with dual functions in lipid metabolism, specifically facilitation of long-chain fatty acid transport across cell membranes and regulation of cytosolic free coenzyme A/acetyl coenzyme A ratio. In the present invention, carnitine is added to a cell culture medium suitable for sustaining the growth of an animal cell line in order to improve the growth of the cell line in the medium. The inventors have studied the effects of carnitine supplementation of different media in a range of concentrations. In all cases, different specimens, such as human skin, spleen, kidney, tumor, amniocyte, and β-lymphocyte cells, were cultured according to classic tissue culture techniques, and controls were set up in parallel utilizing the same conditions for each sample with medium not containing carnitine. Numerous advantageous effects were observed in the carnitine-supplemented media. These effects are referred to generally in this specification as better growth, as this term is descriptive in general of all the specific effects. In some cases faster cell growth was seen; in other cases, initiation of better quality subcultures was seen. Chromosomal studies show no alterations after carnitine supplementation of the tissue culture media. Accordingly, L-carnitine appears to be a useful supplement for tissue culture media and animal cell lines of all types.

L-carnitine does not appear to have been utilized in tissue culture media in the published literature. There is also no listing of L-carnitine in the following media available commercially in the United States at this time: Eagle's medium, Earle's medium, BGJ8 medium, CMRL 1066 medium, defined medium A2+APG, Dulbecco's modified Eagle medium, Iscove's modified Dulbecco's medium, Fischer's medium for leukemia cells of mice, Grace's insect culture media, L15 (Leibovitz) medium, Marx M14-M20 media, McCoy's medium, medium 199, minimum essential medium (MEM), α-MEM, NCTC135 medium, RPMI1603, RPMI1634, RPMI1640, Ham's F10 medium, Ham's F12 medium, Ham's F12M medium, Ham's F12K medium, MCBD101-104 media, Neumann and Tytoll serumless medium, Schneider's drosophyla medium, Swim's 67G medium, Swim's S 77 medium, Trowell T8 medium, Waymouth's medium, William's medium, BME media series, and Chang's medium.

The method of operation of carnitine in cell culture media is not known. In many cases supplemental carnitine does not appear to be needed for minimal cell growth. However, the addition of carnitine results in better cell growth as described above. The enhancement of both rate and quality of growth has been experimentally demonstrated by adding carnitine to a number of cell culture media previously though to be complete. These include media most commonly used in tissue culture laboratories, such as Difco blood kit, RPMI 1640, MEM, α-MEM, and Chang's medium. A wide variety of explants and other cell sources, including human skin, spleen, kidney, tumor, amniocyte, and B-lymphocyte cells, have been shown to be advantageously affected by the addition of carnitine to a cell culture medium in which the cells were growing.

The amount of added carnitine for a particular cell line can be adjusted by one skilled in the art to find either the minimum amount sufficient to improve the growth of the cell line or the optimum amount. Generally one begins with a "complete" cell culture medium considered suitable for sustaining the growth of the animal cell line being cultivated. A number of published reports and general catalogs, such as those described above, describe such cell culture media. A number of these media are available commercially. Grand Island Biological Company (GIBCO) is a well known commercial supplier of media. The latest edition of the GIBCO catalog can be consulted both to determine commercially available cell culture media and to define the composition of such media.

L-carnitine is commercially available from Sigma-Tau, an Italian supplier, or can be synthesized according to the method described by Tomita and Sindju, J. Physiol. Chem. (1927) 169:263. Carnitine is added to a complete cell culture medium initially in an amount in the range from 0.02 to 5 percent by weight, preferably 0.10 to 2.0 percent by weight (g of carnitine per 100 ml of medium). Improved cell growth can be optimized by adjusting the amount of added carnitine up or down from the initial amount added. When minimum essential medium with 20% fetal calf serum and 2% L-glutamine is utilized, 0.2% by weight of carnitine has proven to be optimal. Cell culture media for which carnitine supplementation should be effective include the following: ATCC-CRCM 30; CMRL 1066 and 1415; Dolbecco's modification of Eagle's medium; Eagle's basal medium (BME); Eagle's minimum essential medium (MEM); α-MEM; Chang's medium; Ham's F10, F12, F12M, F12K, and MCDB 101-104; Leibovitz's L-15; McCoy's 5A; medium 199; NCTC 109 and 135; Puck's N15 and N16; RPMI 1603, 1634 and 1640; and Waymouth's MB 752/1. Other cell culture media, either not known or later discovered, capable of sustaining the growth of an animal cell line can be improved by incorporating L-carnitine into the medium as described in this specification.

Although any animal cell line can be utilized with a medium of the invention, carnitine is particularly suitable for the supplementation of culture media utilized with cells obtained from explants (solid tissue specimens) or with hybridomas. Cell lines derived from humans, particularly skin, spleen, kidney, tumor, amniocyte, and beta-lymphocyte-derived cell lines are particularly preferred for practice with the present invention.

The invention now being generally understood, the following examples are set forth to provide exemplification of specific embodiments of the invention. These examples are not to be considered limiting of the invention unless otherwise stated.

## EXAMPLES

The effects of L-carnitine supplementation of different media (Difco blood kit, RPMI 1640, MEM, α-MEM, and Chang media) in concentrations ranging from 50mg%-2g% were studied. The specimens (human skin, spleen, kidney, tumor, amniocyte, and beta-lymphocyte cells) were cultured according to classic tissue culture techniques, and controls were set up in parallel utilizing the same conditions for each sample with each medium but not containing carnitine.

## Cell Lines

Solid tissue specimens were obtained from surgery, biopsy, or autopsy materials consisting of a section of the indicated tissue. Aseptic techniques were utilized for obtaining 2x2mm pieces. These pieces were adhered to the bottom of culture dishes (plastic Corning petri 100/50mm tissue-culture dish). Culturing eventually resulted in fibroblast outgrowth. The specimen was then cut with a sterile scalpel into 2x2mm explants which were positioned on an X-scratched area on the bottom of a culture dish. Then 0.5ml of the indicated medium was pipetted over each explant (4-8 explants per dish). The cultures were then incubated in a humidified incubator (95% relative humidity, 5% $CO_2$) for 24-48 hours. Then 5ml of medium were added to each culture and changed every other day for the length of the culture. Cultures were checked daily and subjectively evaluated for initiation and amount of fibroblast growth using a Swift inverted microscope.

## Amniotic Cell Culture

Amniotic cell culture was initiated from specimens of 15-30ml of amniotic fluid from patients during the 16-20th week of gestation. Optimal number of cells for genetic studies were obtained by long-term culture of amniotic cells that were separated from the amniotic fluid by centrifugation. Falcon 35mm petri dishes with a 22mm² microscope cover slip were used for culture. Using sterile techniques, the centrifuged cell button was resuspended in 1-2ml medium, and 0.5ml of the suspended cells was pipetted onto the cover slip in the dish. The cultures were maintained in a humidified incubator (95% relative humidity, 5% $CO_2$, 37°C). After the first 24 hours, 1.5ml of warm medium was added, and medium was then changed every other day. Culture dishes were checked and evaluated daily using a Swift inverted microscope. The total number of colonies was counted for each cell line and medium, and each colony was measured for surface area.

## Results

Statistical analysis of forty amniocyte cultures set up in parallel with controls showed that cultures supplemented with L-carnitine contained more cells at seven days after initiation in an amount that was significant at the 10% level ($p > 0.1$; matched-pair analysis using t-test).

No attempt was made in either the solid explant or amniocyte studies to optimize the amount of carnitine added to the individual samples. Accordingly, statistically significant improved growth was not seen in all individual cases. However, 50% of the fifty-three amniotric cultures grew better in the L-carnitine supplemented media (better here referring to better plating and cloning efficiency). No culture failures appeared in the twenty-eight solid tissue cultures, 57% of the solid tissue cultures showed a better growth rate, and 53% of the solid tissue cultures demonstrated better initiation than the corresponding controls. In eleven of the samples, there was a time delay of 24-72 hours between set up and culture. In all of these cases, faster and better growth was seen in the L-carnitine supplemented media.

The individual results are set forth in the two tables below. The first of these tables demonstrates advantageous effects of carnitine on a qualitative basis for a number of different types of cell lines and media. Table 2 shows a statistical analysis of forty amniocyte cultures in α-MEM to demonstrate the statistically significant effect of carnitine on growth.

## TABLE 1

### Tissue Culture with L-carnitine Supplemented Media

| Lab No.[1] | # Samples Carn[2] | Contr | Time Delay for Set-up (hours) | Days of Culture for First Cells Carn | Contr | Days of Culture for Subculture Carn | Contr | Carnitine Concentration g% | Observations | Concl[5] |
|---|---|---|---|---|---|---|---|---|---|---|
| 040 | 2 | 3 | 16 | 4 | 4 | 10 | 12 | 1.0 | Better growth with C | Better |
| 041 | 2 | 3 | 72 | 3 | 4 | 8 | 10 | 0.5/1.0 | Sample w/0.5% better ~ 1.0% | Better |
| 042 | 1 | 4 | 0 | 4 | 4 | ?[4] | 10 | 1.0 | Spleen cells, control better | Worse |
| 043 | 2 | 3 | 0 | 3 | 4 | 14 | 14 | 0.5/1.0 | Sample w/0.5% better ~ 1.0% | Better |
| 044 | 2 | 2 | 16 | 2 | 2/5 | 8 | 8 | 1.0 | Kidney and tumor tissue | Better |
| 045 | 2 | 2 | 0/48[3] | 1/2 | 1/2 | 8/? | 8/? | 1.0 | Fetus, time delay set not subcultured, better | Better |
| 046 | 2 | 2 | 0/48 | 1/4* | 1/4 | 7/? | 7/? | 1.0 | Fetus,* initiation better in quality | Same |
| 047 | 2 | 2 | 0/48 | 1/3* | 1/3 | 6/? | 6/? | 1.0 | Fetus,* initiation better in quality | Same |
| 048 | 1 | 1 | 0 | 4* | 4 | 7 | ? | 1.0 | All explants grew with C, initiation better | Better |
| 049 | 2 | 2 | 0/24 | 3/3 | 4/4 | 11/11 | 11/11 | 1.0 | Same; time delay better with C | Same/Better |

0 263 659

TABLE 1 (Cont'd)

Tissue Culture with L-carnitine Supplemented Media

| Lab No.[1] | # Samples[2] | | Time Delay for Set-up (hours) | Days of Culture for First Cells | | Days of Culture for Subculture | | Carnitine Concentration g% | Observations | Concl[5] |
|---|---|---|---|---|---|---|---|---|---|---|
| | Carn | Contr | | Carn | Contr | Carn | Contr | | | |
| 050 | 1 | 1 | 24 | 2 | 2 | ? | ? | 1.0 | At 6 days better with C | Better |
| 051 | 1 | 1 | 48 | 1 | 2 | ? | ? | 0.5 | At 5 days better with C | Better |
| 054 | 1 | 1 | 24 | 4 | 4 | 7 | ? | 0.2 | Much better, all explants growing | Better |
| 038 | 2 | 3 | 0 | 3 | 3 | 14 | 14 | 1 | Much better with C, bigger colonies | Better |
| 039 | 2 | 3 | 0 | 1 | 2 | 5 | 7 | 1 | | Better |
| 037 | 2 | 3 | 0 | 1 | 1 | 6 | 6 | 0.1 | Fetus, same growth | Same |
| 036 | 2 | 3 | 0 | 3 | 5 | 10 | 13 | 0.1 | | Better |

NOTES:

1  Lab No. identifies the patient from whom the samples were taken. All cultured cells were skin fibroblasts (unless otherwise indicated under the heading observations, in which case they were fibroblasts from the indicated source). All samples were cultured in MEM.

2  CARN and CONTR refer to carnitine-supplemented and control samples, respectively.

3  A slash (/) separates two samples for the same lab number that were treated differently.

4  "?" means culture was discarded befoe it was ready for subculture.

5  Conclusion was reached based on both quality and quantity of cells growing from primary explants, total number of explants growing, and the time necessary for first cells and subculture. "Better" indicates better growth for the carnitine-containing sample.

0 263 659

## TABLE 2

### Statistical Analysis of Amniocyte Cultures Matched-Pair Analysis – t Test

| Sample Number | C | Co | D | $(D-\bar{D})$ | $(D-\bar{D})^2$ |
|---|---|---|---|---|---|
| 1 | 2 | 8 | -6 | -8.2 | 67.24 |
| 2 | 2 | 5 | -3 | -5.2 | 27.04 |
| 3 | 3 | 2 | +1 | -1.2 | 1.44 |
| 4 | 1 | 2 | -1 | -3.2 | 10.24 |
| 5 | 16 | 10 | +6 | +3.8 | 14.44 |
| 6 | 2 | 1.5 | +0.5 | -1.7 | 2.89 |
| 7 | 8 | 4 | +4 | +1.8 | 3.24 |
| 8 | 32 | 32 | 0 | -2.2 | 4.84 |
| 9 | 1 | 2 | -1 | -3.2 | 10.24 |
| 10 | 24 | 5 | +19 | +16.8 | 282.24 |
| 11 | 16 | 2 | +14 | +11.8 | 139.24 |
| 12 | 2 | 0.5 | +1.5 | -0.7 | 0.49 |
| 13 | 14 | 1.5 | +12.5 | +10.3 | 106.09 |
| 14 | 0 | 5 | -5 | -7.2 | 51.84 |
| 15 | 4 | 10 | -6 | -8.2 | 67.24 |
| 16 | 6 | 5 | +1 | -1.2 | 1.44 |
| 17 | 10 | 3 | +7 | +4.8 | 23.04 |
| 18 | 20 | 10 | +10 | +7.8 | 60.84 |
| 19 | 6 | 1 | +5 | +2.8 | 7.84 |
| 20 | 3 | 3 | 0 | -2.2 | 4.84 |
| 21 | 40 | 36 | +4 | +1.8 | 3.24 |
| 22 | 36 | 36 | 0 | -2.2 | 4.84 |
| 23 | 8 | 5 | +3 | +0.8 | 0.64 |
| 24 | 4 | 4 | 0 | -2.2 | 4.84 |
| 25 | 7 | 6 | +1 | -1.2 | 1.44 |
| 26 | 24 | 20 | +4 | +1.8 | 3.24 |
| 27 | 26 | 9 | +17 | +14.8 | 219.04 |
| 28 | 1 | 10 | -9 | -11.2 | 125.44 |
| 29 | 10 | 10 | 0 | -2.2 | 4.84 |
| 30 | 18 | 8 | +10 | +7.8 | 60.84 |
| 31 | 8 | 5 | +3 | +0.8 | 0.64 |
| 32 | 3 | 4 | -1 | -3.2 | 10.24 |
| 33 | 32 | 16 | +16 | +13.8 | 190.44 |
| 34 | 10 | 8 | +2 | -0.2 | 0.04 |
| 35 | 4 | 26 | -22 | -24.2 | 585.64 |
| 36 | 14 | 26 | -12 | -14.2 | 201.64 |
| 37 | 6 | 26 | -20 | -22.2 | 492.84 |
| 38 | 12 | 10 | +2 | -0.2 | 0.04 |
| 39 | 30 | 10 | +20 | +17.8 | 316.84 |
| 40 | 20 | 10 | +10 | +7.8 | 60.84 |

$\Sigma D = 87.5$    $\bar{D} = 2.18$    $\Sigma(D-\bar{D})^2 = 3170.32$    $S_D = 9.01$    $S_{\bar{D}} = 9.01/6.32 = 1.42$

$t = \bar{D}-0/S_{\bar{D}} = 2.18/1.42 = 1.53$

* Values for C ∝ Co (carnitine-containing sample and control sample, respectively) are the sum of colony diameters (mm) at 7 days of amniocyte cultures. The statistical significance is expected to be much higher if cells are counted in each individual colony rather than expressing as colony diameters. Colony diameter increases arithmetically while cell number in a particular colony increases logarithmically.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto.

## Claims

1. In a cell culture medium suitable for sustaining the growth of an animal cell line, an improvement which comprises:

L-carnitine incorporated in said medium in an amount sufficient to improve the growth of said cell line.

2. The cell culture medium of Claim 1, wherein said amount is from 0.02 to 5% by weight.

3. The cell culture medium of Claim 1 or Claim 2, wherein said amount is from 0.10 to 2.0% by weight.

4. The cell culture medium of any one of the preceding Claims, wherein said medium is ATCC-CRCM 30; CMRL 1066 and 1415; Dulbecco's modification of Eagle's medium: Eagle's basal medium (BME); Eagle's minimum essential medium (MEM); α-MEM; Chang's medium; Ham's F10, F12, F12M, or F12K, MCDB 101, 102, 103, or 104; Leibovitz's L-15; McCoy's 5A; Medium 199; NCTC 109 or 135; Puck's N15 or N16; RPMI 1603, 1634, or 1640; or Waymouth's MB 752/1.

5. The cell culture medium according to any one of Claims 1 to 3, wherein said medium is Minimum Essential Medium.

6. The cell culture medium of any one of the preceding Claims, wherein said cell line is a human cell line obtained from a normal human cell.

7. The cell culture medium of any one of Claims 1 to 5, wherein said cell line is a hybridoma cell line.

8. A method of improving the growth of an animal cell line in a cell culture medium, which comprises:

adding to a cell culture of said cell line in said medium an amount of L-carnitine sufficient to improve the growth of said cell line.

9. The method of Claim 8, wherein said amount is from 0.02 to 5% by weight.

10. The method of Claim 8 or Claim 9, wherein said amount is from 0.10 to 2.0% by weight.

11. The method of Claim 8, Claim 9 or Claim 10, wherein said medium is ATCC-CRCM 30; CMRL 1066 and 1415; Dulbecco's modification of Eagle's medium; Eagle's basal medium (BME); Eagle's minimum essential medium (MEM); α-MEM; Chang's medium; Ham's F10, F12, F12M, or F12K; MCDB 101, 102, 103, or 104; Leibovitz's L-15; McCoy's 5A; Medium 199; NCTC 109 or 135; Puck's N15 or N16; RPMI 1603, 1634 or 1640; or Waymouth's MB 752/1.

12. The method of Claim 8, Claim 9 or Claim 10, wherein said medium is minimum essential medium.

13. The method of any one of Claims 8 to 12, wherein said cell line is a human cell line obtained from a normal human cell.

14. The method of any one of Claims 8 to 12, wherein said cell line is a hybridoma cell line.

15. The method of any one of Claims 8 to 14, wherein said adding takes place from 12 to 72 hours after said cell line is added to said medium.